(19) Europäisches Patentamt
European Patent Office
Office européen des brevets

(11) Publication number: **0 060 951 A1**

# (12) EUROPEAN PATENT APPLICATION

(21) Application number: 81305139.8

(22) Date of filing: 29.10.81

(51) Int. Cl.[3]: C 07 C 87/62
C 07 C 121/78, A 01 N 33/18
A 01 N 37/34

(30) Priority: 19.03.81 US 245424

(43) Date of publication of application: 29.09.82 Bulletin 82/39

(84) Designated Contracting States: AT BE CH DE FR GB IT LI LU NL SE

(71) Applicant: ELI LILLY AND COMPANY
307, East McCarty Street
Indianapolis Indiana 46285(US)

(72) Inventor: Dreikorn, Barry Allen
9731 Trilobi Drive
Indianapolis Indiana 46236(US)

(72) Inventor: Kramer, Kenneth Edward
6455, North Oakland Avenue
Indianapolis Indiana 46220(US)

(74) Representative: Crowther, Terence Roger et al,
Erl Wood Manor
Windlesham Surrey GU20 6PH(GB)

## (54) Improvements in or relating to N-alkylated diphenylamine derivatives.

(57) Diphenylamines of formula (I):

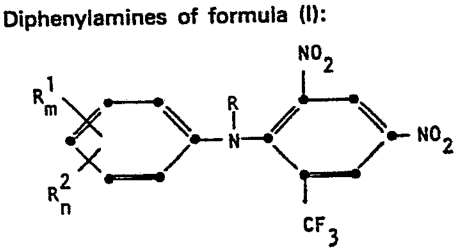

(1)

wherein

R is methyl, ethyl, or n-propyl; each $R^1$ is independently bromo, chloro, or fluoro;
$R^2$ is iodo, cyano, nitro, or trifluoromethyl;
m is an integer of from 0 to 5;
n is an integer of from 0 to 1;
and the sum of m and n is an integer
(1) when n is 0, from 1 to 5; and
(2) when n is 1, from 1 to 3 subject to the further limitations that (1) not more than two $R^1$ groups represent bromo; (2) where $R^2$ is iodo, the iodo is located at the 4-position; (3) where $R^2$ is cyano, the cyano is located at the 3-, 4-, or 5-position; and (4) where $R^2$ is $NO_2$, the $NO_2$ is located at the 2-, 3-, 5-, or 6-position, are effective in controlling insects, arachnids and ectoparasites.

EP 0 060 951 A1

COMPLETE DOCUMENT

# IMPROVEMENTS IN OR RELATING TO N-ALKYLATED DIPHENYLAMINE DERIVATIVES

This invention relates to certain N-alkylated diphenylamine derivatives which are useful in controlling insects, arachnids and ectoparasites.

U.S. Patent Specification No. 4,117,167 discloses compounds similar to those of the present invention except that in that document the central nitrogen atom of the diphenylamine system is not alkylated.

It has now been discovered that alkylation of that central nitrogen atom dramatically reduces the phytotoxicity of this class of compounds, and thus renders them useful as pesticides in areas of economically important plant cultivation.

Thus, in accordance with the invention, diphenylamine derivatives of Formula (I):

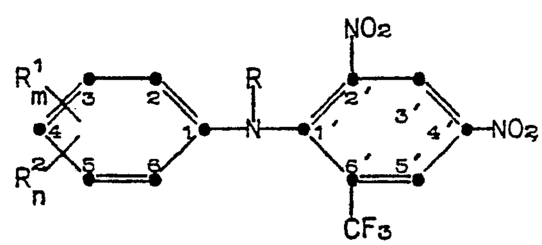

(I)

wherein R = methyl, ethyl, or n-propyl;

each $R^1$ is independently bromo, chloro, or fluoro;

$R^2$ is iodo, cyano, nitro, or trifluoromethyl;

m is an integer of from 0 to 5;

n is an integer of from 0 to 1;

and the sum of m and n is an integer

(1) when n is 0, from 1 to 5; and

(2) when n is 1, from 1 to 3

subject to the further limitations that (1) not more than two $R^1$ groups represent bromo; (2) where $R^2$ = iodo, the iodo is located at the 4-position; (3) where $R^2$ = cyano, the cyano is located at the 3-, 4-, or 5-position; and (4) where $R^2$ = $NO_2$, the $NO_2$ is located at the 2-, 3-, 5-, or 6-position, are useful in controlling insects, arachnids and ectoparasites.

Certain diphenylamines of formula (I) are novel, these compounds have the formula (II)

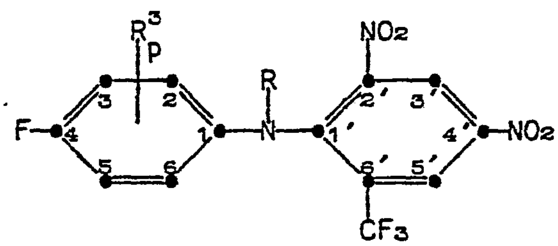

(II)

wherein R is, as above, methyl, ethyl, or n-propyl; and $R^3_p$ is one of the following:

1) p = 0;
2) p = 1 and $R^3$ = 2-F, 3-F, 2-Cl, or 2-Br;
3) p = 2 and $R^3$ = 2-F-6-Br or 2-F-6-Cl; or
4) p = 2, 3, or 4 and each $R^3$ = F.

The novel compounds of the present invention include the following:

N-methyl-, ethyl-, or n-propyl-4-fluoro-2',4'-dinitro-6'-(trifluoromethyl)diphenylamine

N-methyl-, ethyl-, or n-propyl-2,4-difluoro-2',4'-dinitro-6'-(trifluoromethyl)diphenylamine

N-methyl-, ethyl-, or n-propyl-3,4-difluoro-2',4'-dinitro-6'-(trifluoromethyl)diphenylamine

N-methyl-, ethyl-, or n-propyl-2-bromo-4-fluoro-2',4'-dinitro-6'-(trifluoromethyl)diphenylamine

N-methyl-, ethyl-, or n-propyl-2-chloro-4-fluoro-2',4'-dinitro-6'-(trifluoromethyl)diphenylamine

N-methyl-, ethyl-, or n-propyl-2,4,6-trifluoro-2',4'-dinitro-6'-(trifluoromethyl)diphenylamine

N-methyl-, ethyl-, or n-propyl-2,3,4-trifluoro-2',4'-dinitro-6'-(trifluoromethyl)diphenylamine

N-methyl-, ethyl-, or n-propyl-2,4,5-trifluoro-2',4'-dinitro-6'-(trifluoromethyl)diphenylamine

N-methyl-, ethyl-, or n-propyl-3,4,5-trifluoro-2',4'-dinitro-6'-(trifluoromethyl)diphenylamine

N-methyl-, ethyl-, or n-propyl-2,4-difluoro-6-bromo-2',4'-dinitro-6'-(trifluoromethyl)diphenylamine

N-methyl-, ethyl-, or n-propyl-2,4-difluoro-6-chloro-2',4'-dinitro-6'-(trifluoromethyl)diphenylamine

N-methyl-, ethyl-, or n-propyl-2,3,4,5-tetrafluoro-2',4'-dinitro-6'-(trifluoromethyl)diphenylamine

N-methyl-, ethyl-, or n-propyl-2,3,4,6-tetrafluoro-2',4'-dinitro-6'-(trifluoromethyl)diphenylamine

N-methyl-, ethyl-, or n-propyl-2,3,4,5,6-pentafluoro-2',4'-dinitro-6'-(trifluoromethyl)diphenylamine

All of the diphenylamines of formula (I) are useful for the control of phytophagous insects and arachnids. Therefore, in one aspect of the invention there is provided a method for inhibiting a phytophagous insect or arachnid which comprises applying to a locus of the insect or arachnid an effective insect- or arachnid- inhibiting amount of a diphenylamine of formula (I).

However, although the present compounds exhibit moderate insecticidal activity, they exhibit a higher order of activity against arachnids, especially the Acarina (mites). Therefore, in a preferred embodiment, the present invention is directed to a method for inhibiting a phytophagous mite which comprises applying to a plant a miticidally effective amount of a diphenylamine in accordance with the present invention.

Representative mite species with which the present invention can be practiced include those listed in the following table.

| Family | Scientific Name | Common Name |
|---|---|---|
| ACARIDAE | | |
| | *Aleurobius farinae* | Flour mite |
| | *Rhizoglyphus echinopus* | Bulb mite |
| | *Rhizoglyphus elongatus* | Elongate mite |
| | *Rhizoglyphus rhizophagus* | Root mite |
| | *Rhizoglyphus sagittatae* | Balsam root mite |
| | *Rhizoglyphus tarsalis* | Beet mite |
| ERIOPHYIDAE | | |
| | *Abacarus hystrix* | |
| | *Aceria brachytarsus* | |
| | *Aceria essigi* | Redberry mite |
| | *Aceria ficus* | |
| | *Aceria fraxinivorus* | |
| | *Aceria granati* | |
| | *Aceria parapopuli* | Cottonwood mite |
| | *Aceria sheldoni* | Citrus bud mite |
| | *Aceria tulipae* | Wheat curl mite |
| | *Aculus schlechtendali* | Apple rust mite |

| | |
|---|---|
| *Eriophyes convolvens* | |
| *Eriophyes insidiosus* | |
| *Eriophyes malifoliae* | Apple leaf mite |
| *Eriophyes padi* | Plum twig gall mite |
| *Eriophyes pruni* | Plum leaf gall mite |
| *Eriophyes pyri* | Pear leaf blister mite |
| *Eriophyes ramosus* | Juniper mite |
| *Eriophyes ribis* | Currant gall mite |
| *Eriophyes vitis* | Grape erineum mite |
| *Phyllocoptes gracilis* | Blackberry mite |
| *Phyllocoptruta oleivora* | Citrus rust mite |
| *Phytoptus avellanae* | Filbert mite |
| *Phytoptus ribis* | |
| *Trisetacus pini* | Pine needle mite |
| *Vasates amygdalina* | David peach mite |
| *Vasates cornutus* | Peach silver mite |
| *Vasates eurynotus* | Celery rust mite |
| *Vasates schlechtendali* | Rusty leaf mite |
| EUPODIDAE | |
| *Linopodes spp.* | |
| PENTHALEIDAE | |
| *Halotydeus destrustor* | Redlegged earth mite<br>Black sand mite |
| *Penthaleus major* | Winter grain mite<br>Blue oat mite |
| PYEMOTIDAE | |
| *Siteroptes cerealium* | |

| | |
|---|---|
| TARSONEMIDAE | |
| *Steneotarsonemus pallidus* | Cyclamen mite |
| TENUIPALPIDAE | |
| *Brevipalpus californicus* | California citrus mite |
| *Brevipalpus obovatus* | |
| *Brevipalpus lewisi* | Flat mite |
| *Tenuipalpes granati* | |
| *Tenuipalpes pacificus* | |
| TETRANYCHIDAE | |
| *Bryobia arborea* | Brown mite |
| *Bryobia rubrioculus* | |
| *Eotetranychus coryli* | |
| *Eotetranychus lewisi* | Lewis spider mite |
| *Eotetranychus sexmaculatus* | Six spotted spider mite |
| *Eotetranychus weldoni* | Weldon's mite |
| *Eotetranychus willametti* | |
| *Eutetranychus banksi* | Texas citrus mite |
| *Mediolata mali* | Apple mite |
| *Oligonychus ilicis* | Southern red mite |
| *Oligonychus pratensis* | Timothy mite, Banks grass mite |
| *Oligonychus ununguis* | Spruce tree spider mite |
| *Panonychus citri* | Citrus red mite |
| *Panonychus ulmi* | European red mite |
| *Paratetranychus modestus* | Corn mite |
| *Paratetranychus pratensis* | Date mite |

| | |
|---|---|
| *Paratetranychus viridis* | Green mite |
| *Petrobia decepta* | Barley mite |
| *Schizotetranychus celarius* | Bamboo mite |
| *Schizotetranychus pratensis* | Alfalfa mite |
| *Tetranychus canadensis* | Four spotted mite |
| *Tetranychus cinnabarinus* | |
| *Tetranychus mcdanieli* | McDaniel mite |
| *Tetranychus pacificus* | Pacific mite |
| *Tetranychus schoenei* | Schoene mite |
| *Tetranychus telarius* | Common red spider |
| *Tetranychus urticae* | Two-spotted spider mite |
| *Tetranychus turkestani* | Strawberry mite |
| *Tetranychus desertorum* | Desert mite |

The diphenylamines of formula (I) are usually formulated with one or more non-phytotoxic carriers or adjuvants such as water, organic solvents, surfactants, inert finely-divided solids, propellants (as in an aerosol), stickers which will insure adherence of a treating formulation to foliage, and the like. The use of a surfactant is generally preferred. Such formulations will generally contain from 0.1 to 95% of the diphenylamine.

The diphenylamine derivatives of formula (I) are conveniently formulated as concentrated compositions which are diluted prior to application, for instance by the addition of water or other suitable

diluent to make a dispersion, emulsion or the like. Typical concentrated formulations which are in the form of solids are wettable powders. Wettable powders comprise a finely-divided intimate mixture of an inert carrier or excipient, a diphenylamine derivative, and a suitable surfactant. Commonly utilized inert carriers which are agronomically acceptable include the diatomaceous earths, the various clays such as attapulgite clays or kaolin clays, or silica. Surfactants generally are present in about 0.5 to about 15 percent by weight of the wettable powder, with the diphenylamine derivative preferably being present in about 10 to about 60 percent by weight. Any of several known surfactants can be employed in the wettable powder formulations, including any of the sulfonated lignins, the condensed naphthalenesulfonates, the alkylbenzenesulfonates, the alkyl sulfates, as well as the nonionic surfactants such as the ethylene oxide adducts of phenols. The wettable powders which are thus comprised generally are diluted with water or the like prior to application. Such formulation is then applied as a spray or the like by conventional sprayers and other agricultural chemical applicators.

Another commonly utilized formulation type to be employed with the diphenylamine derivative are the emulsifiable concentrates. Such formulations are comprised of a diphenylamine admixed with a carrier such as a water immiscible organic solvent and an emulsifying agent, in an amount from 0.5 to 15 percent by weight of the emulsifiable concentrate. Water

miscible cosolvents may be utilized in conjunction with the immiscible solvent to improve solubility of the diphenylamine. Commonly used solvents include toluene, xylene, chlorotoluene, benzene, methyl isobutyl ketone, cyclohexanone, naphtha and the like. Aqueous suspensions employing water as a diluent also are contemplated. Emulsifiers which can be employed include the common surfactants and blends of surfactants, for example the alkyl and aryl sulfonates, ethoxylated alkyl phenols, ethoxylated alkyl ethers, the nonoxynols, oxysorbics, allinols, allinates and other nonionic and anionic surfactants. The surfactant may comprise from 0.5 to 10 percent by weight of the suspension. Such emulsifiable concentrates, like the wettable powder formulations, are diluted prior to application, for instance by the addition of the appropriate amount of water to provide a mixture having the desired concentration of active ingredient.

The actual amount of compound to be applied to loci of insects and mites is not critical and can readily be determined by those skilled in the art in view of the examples below. In general, concentrations of from 10 ppm to 5,000 ppm of compound are expected to provide good control. With many of the compounds, concentrations of from 100 to 1,500 ppm will suffice. For field crops such as soybeans and cotton, a suitable application rate for the two most preferred compounds (defined below) is about 0.5 to 1.5 lbs./acre, typically applied in 50 gallons per acre of spray formulation containing 1200 to 3600 ppm of compound.

The locus to which a compound is applied can be any locus inhabited by a phytophagous insect or arachnid, for example, vegetable crops, fruit and nut trees, grape vines, and ornamental plants. Inasmuch as many mite species are specific to a particular host, the foregoing list of mite species also provides exemplification of the wide range of settings in which the present compounds can be used.

Because of the unique ability of mite eggs to resist toxicant action, repeated applications may be desirable to control newly emerged larvae, as is true of other known acaricides.

As insecticides and arachnicides generally, and as acaricides in particular, certain of the present diphenylamines are preferred. Preferred compounds are those wherein R = methyl or ethyl, and especially those wherein R = methyl. Preferred substitution patterns on the

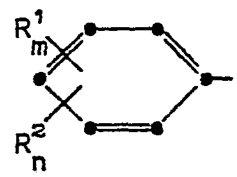

ring include 3-(trifluoromethyl), 4-fluoro, 4-bromo, 4-chloro, 2,4-difluoro, 2-chloro-4-fluoro, and 2-bromo-4-fluoro. The most preferred compounds appear to be N-methyl-3-(trifluoromethyl)-2',4'-dinitro-6'-(trifluoromethyl)diphenylamine and N-methyl-2,4-difluoro-2',4'-dinitro-6'-(trifluoromethyl)diphenylamine, and of these two, especially the latter.

The diphenylamines of the invention can be prepared by known means, see for example, U.S. Patents Specification No. 4,187,318. In general, the compounds are prepared by one of the following reaction routes:

I. Condensation followed by alkylation:

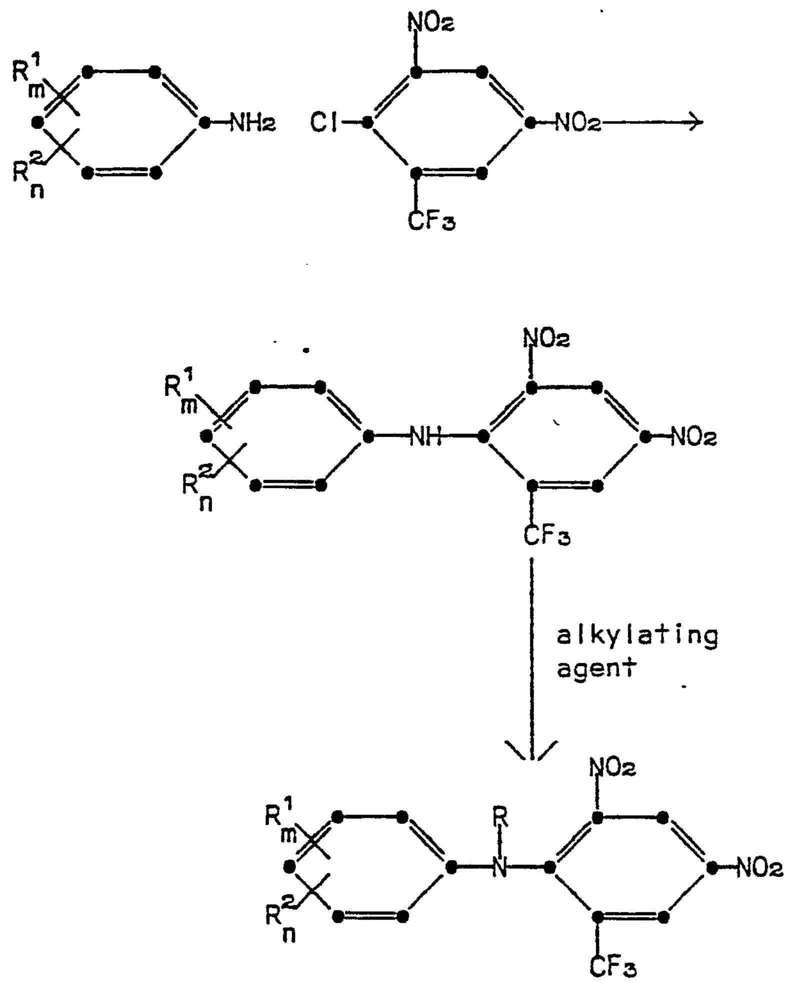


The reaction between the aniline derivative and the halophenyl compound is a simple amination which can be carried out using techniques well known to those skilled in the art. An acid scavenger, such as an

inorganic base, a tertiary amine or excess of the aniline derivative is needed in the reaction mixture. In general, the condensation is most conveniently effected using dimethylformamide as solvent, at a temperature of about -10°C, and using sodium hydride as the acid scavenger. The NH-product is then alkylated by generating the anion with a weak base, preferably sodium carbonate, followed by reaction with a suitable alkylating agent, preferably a dialkylsulphate such as dimethylsulphate or an alkyl halide such as methyl iodide. The temperature of the alkylation preferably lies in the range from 20 to 120°C. Suitable inert organic solvents for the alkylation include acetone, dimethylformamide and tetrahydrofuran.

II. Halogenation of an N-alkyldiphenylamine (for compounds where one or more $R^1$ is bromo or chloro):

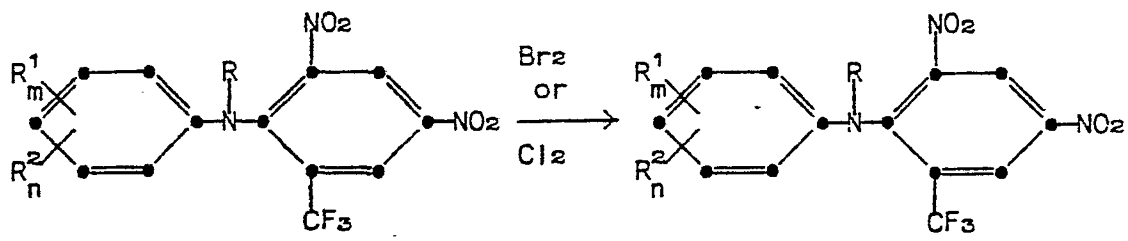

III. Nitration of an N-alkyldiphenylamine (where $R^2$ is $NO_2$):

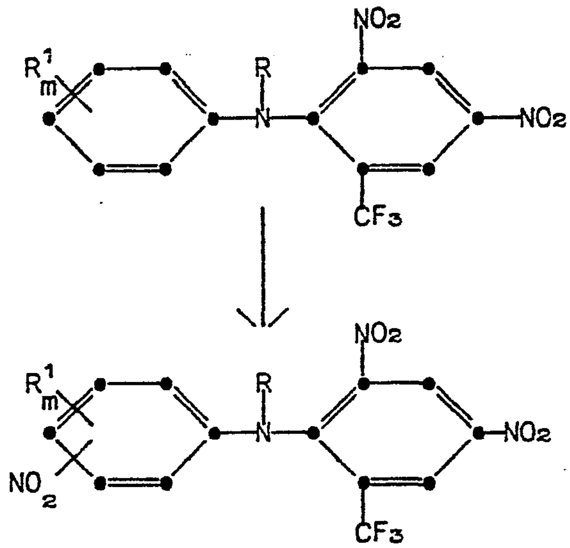

IV. Condensation of a 2-desnitrochlorobenzene and an N-alkylaniline, or an aniline with subsequent alkylation, followed by nitration of the 2'-position (for polyhalo-substituted compounds such as 2,4,6-trihalo-, tetrahalo-, and pentahalo-substituted compounds):

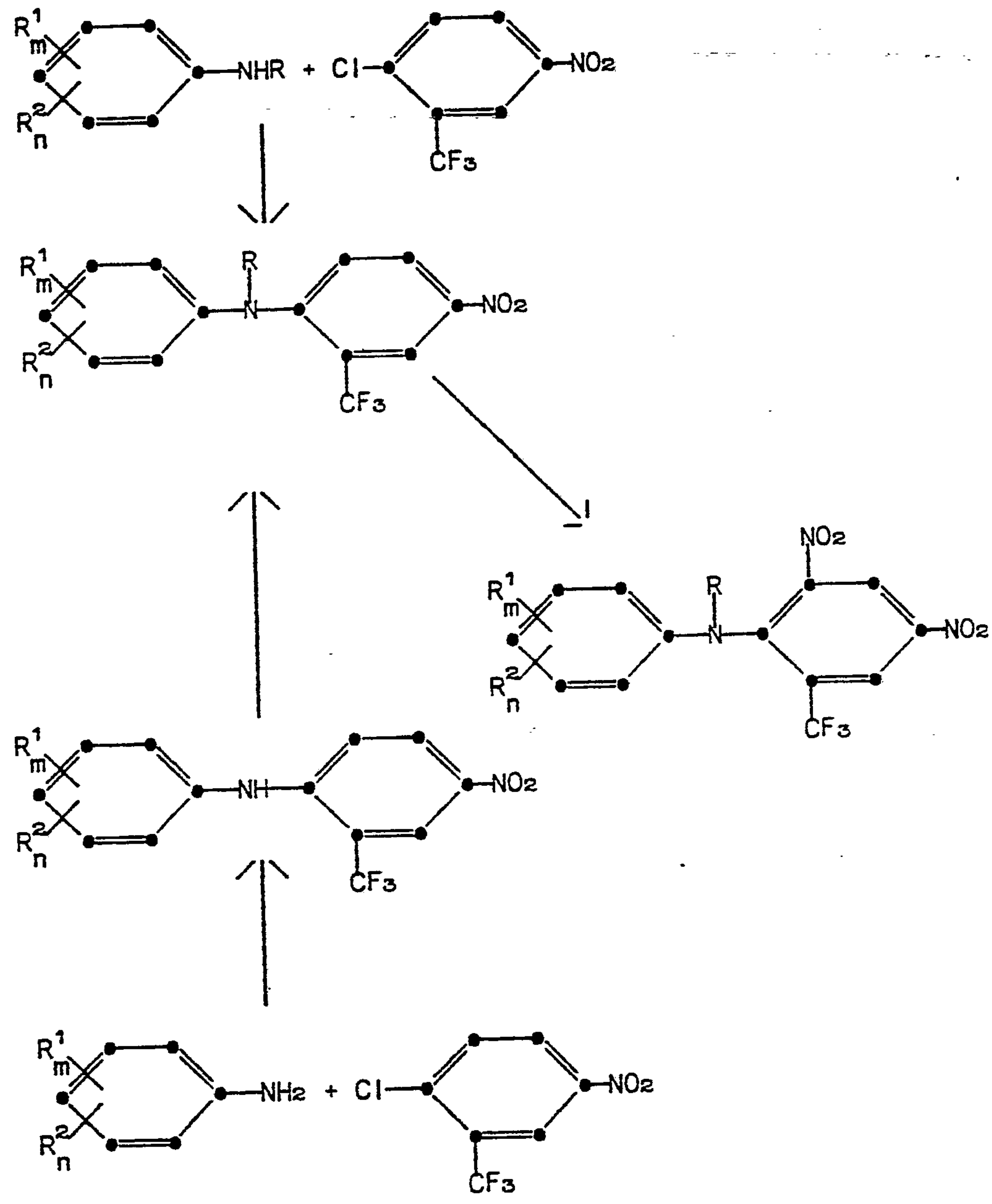

The following examples illustrate the preparation of the compounds to be employed in accordance with the present invention.

EXAMPLE 1: 4-FLUORO-2',4'-DINITRO-6'-(TRIFLUOROMETHYL)DIPHENYLAMINE

4-Fluoroaniline (10.0 grams; 0.09 mole) was combined with 100 ml. of anhydrous ethanol. 1-Chloro-2,4-dinitro-6-(trifluoromethyl)benzene (12.2 grams; 0.045 mole) was added to the resulting solution, and the resulting reaction mixture was refluxed overnight (about 20 hours) under nitrogen. TLC indicated that the reaction had gone to completion. On cooling, a precipitate formed. It was separated by filtration and recrystallized from 75 ml. of ethanol. The identity of the product was confirmed by NMR, IR, and elemental analysis which showed

Calculated for $C_{13}H_7F_4N_3O_4$: C, 45.23; H, 2.04; N, 12.17.
Found: C, 45.29; H, 2.16; N, 12.21.

The total yield (two crops) was 11.9 grams (76.7%). The product melted at 135-136°C.

EXAMPLE 2: N-METHYL-4-FLUORO-2',4'-DINITRO-6'-(TRIFLUOROMETHYL)DIPHENYLAMINE

4-Fluoro-2',4'-dinitro-6'-(trifluoromethyl)-diphenylamine (6.0 grams; 0.017 mole), acetone (50 ml.), sodium carbonate (16.4 grams), and dimethyl sulfate (16.4 ml.) were combined. The resulting reaction mixture was refluxed for 24 hours. TLC showed product plus starting material.

The reaction mixture was then worked up as follows. Water (100 ml.) was added and the reaction

mixture heated for an additional hour. Additional water (200 ml.) was added and the mixture was allowed to cool and stand over a week-end (about 72 hours). The aqueous layer was then decanted off. The oily residue was dried by dissolving it in methylene chloride, treating the solution with anhydrous sodium sulfate, filtering, and stripping off the methylene chloride. The resulting material was purified by passing it through a silica gel column using toluene as eluent. The product fraction was collected and volatile material stripped off. The identity of the product was confirmed by NMR, IR, and elemental analysis, which showed

Calculated for $C_{14}H_9F_4N_3O_4$: C, 46.81; H, 2.53; N, 11.70.
Found: C, 47.00; H, 2.50; N, 11.71.

The product melted at 77-78°C. and the yield was 4.53 grams (72.5%).

EXAMPLE 3: N-METHYL-2,4-DIFLUORO-2',4'-DINITRO-6'-(TRIFLUOROMETHYL)DIPHENYLAMINE

2,4-Difluoro-2',4'-dinitro-6'-(trifluoromethyl)-diphenylamine (2.6 grams; 0.0072 mole), acetone (18 ml.), sodium carbonate (6.8 grams), and dimethyl sulfate (6.8 ml.) were mixed. The reaction mixture was refluxed for four (4) hours. TLC indicated that the reaction had virtually gone to completion. Water (50 ml.) was added and the reaction mixture was heated for one hour. Additional water (100 ml.) was added and the reaction mixture was allowed to stir overnight (about 20 hours) at room temperature. The aqueous layer was then decanted off from the oil. Recrystallization of the oil from ethanol was unsuccessful, and the product was

purified using a silica gel column, with toluene as the eluant. The identity of the product was confirmed by NMR and elemental analysis, which showed the following

Calculated for $C_{14}H_8F_5N_3O_4$: C, 44.58; H, 2.14; N, 11.14.
Found: C, 44.34; H, 2.36; N, 11.16.

The product melted at 108-109°C. and the yield was 0.82 gram (30.2%).

EXAMPLE 4: N-METHYL-2,4,6-TRIFLUORO-2',4'-DINITRO-6'-(TRIFLUOROMETHYL)DIPHENYLAMINE

2,4,6-Trifluoro-2',4'-dinitro-6'-(trifluoromethyl)diphenylamine (5.0 grams; 0.0131 mole), acetone (50 ml.), sodium carbonate (13.0 grams), and dimethyl sulfate (13 ml.) were mixed and refluxed for 24 hours. TLC indicated that there was still some unreacted starting material. The reaction mixture was then refluxed another 24 hours, with stirring. Water (200 ml.) was added, the reaction mixture heated for an additional hour, and another 200 ml. of water was added. The reaction mixture was permitted to cool and was left standing at room temperature for approximately 20 hours. The aqueous mixture was then decanted off from the oil. The oil was dissolved in methylene chloride, treated with anhydrous sodium sulfate and filtered, and the volatile components were stripped off.

The product was recrystallized from ethanol. Its identity was confirmed by NMR and elemental analysis, which showed the following

Calculated for $C_{14}H_7F_6N_3O_4$: C, 42.55; H, 1.79; N, 10.63.
Found: C, 42.30; H, 2.00; N, 10.45.

The product melted at 106-108°C. and the yield was 1.50 grams (29.0%).

EXAMPLE 5: N-METHYL-2-BROMO-4-FLUORO-2',4'-DINITRO-6'-(TRIFLUOROMETHYL)DIPHENYLAMINE

N-Methyl-4-fluoro-2',4'-dinitro-6'-(trifluoromethyl)diphenylamine (2.0 grams; 0.0056 mole), acetic acid (30 ml.), and bromine (1 ml.) were mixed. The reaction mixture was allowed to stir overnight (about 20 hours) at room temperature. TLC indicated that no reaction had taken place. Additional bromine (1 ml.) was added and the reaction mixture was refluxed for one hour. TLC again indicated that no reaction had taken place. Additional bromine (1 ml.) was added and the reaction mixture was refluxed for another three (3) hours, at which time TLC indicated that reaction had taken place. The reaction mixture was then permitted to stand over a weekend (about 72 hours) at room temperature.

Water was added dropwise to fill the reaction flask (250 ml. size); the product precipitated and was separated by filtration and recrystallized from ethanol. The product melted at 160-161.5°C. Its identity was confirmed by NMR and elemental analysis, whcih showed the following

Calculated for $C_{14}H_8BrF_4N_3O_4$: C, 38.38; H, 1.84; N, 9.56.
Found: C, 38.12; H, 2.05; N, 9.60.

The yield was 0.88 gram (36.1%).

EXAMPLE 6: N-METHYL-2-NITRO-4-FLUORO-2',4'-DINITRO-6'-(TRIFLUOROMETHYL)DIPHENYLAMINE

N-methyl-4-fluoro-2',4'-dinitro-6'-(trifluoromethyl)diphenylamine (3.0 grams; 0.0084 mole) was dissolved in 50 ml. of trifluoroacetic acid. To this solution there was added by means of a dropping funnel, 0.60 gram (0.0075 mole) of ammonium nitrate dissolved in 15 ml. of trifluoroacetic acid. The reaction mixture was allowed to stir overnight (about 20 hours) at room temperature. Water was then added until the reaction flask (a 250 ml. size) was full. The product precipitated and was separated by filtration and recrystallized from ethanol. The product melted at 130.5-132°C. Its identity was confirmed by NMR and elemental analysis, which showed the following

Calculated for $C_{14}H_8F_4N_4O_6$: C, 41.60; H, 1.99; N, 13.86.
Found: C, 41.59; H, 1.77; N, 13.71.

The yield was 0.85 gram (25.0%).

EXAMPLE 7: N-ETHYL-2,4-DINITRO-6-(TRIFLUOROMETHYL)-DIPHENYLAMINE

2,4-Dinitro-6-(trifluoromethyl)diphenylamine (14.4 grams; 0.044 mole), acetone (435 ml.), sodium carbonate (57.8 grams), and ethyl iodide (32 ml.) were combined and refluxed for a total of 4 days. The reaction was followed by TLC. After the first day, an additional 10 ml. of ethyl iodide was added. After the second day, an additional 10 ml. of ethyl iodide and 16 grams of sodium carbonate were added. After the third day, an additional 10 ml. of ethyl iodide and 15 grams of potassium carbonate were added.

At the end of the reflux period, the reaction mixture was allowed to cool, the precipitate was removed and discarded, and the filtrate was evaporated. The residue was separated into fractions on an HPLC. The identity of the product fraction was confirmed by NMR.

EXAMPLE 8: N-ETHYL-2,4,6-TRICHLORO-2',4'-DINITRO-6'-(TRIFLUOROMETHYL)DIPHENYLAMINE

N-Ethyl-2,4-dinitro-6-(trifluoromethyl)-diphenylamine (1.2 grams; 0.0034 mole) was dissolved in 100 ml. of acetic acid. Chlorine was bubbled in for two hours as the reaction mixture was refluxed. The reaction mixture was allowed to cool and the acetic acid removed by evaporation. The residue was taken up in methylene chloride and washed with a saturated aqueous solution of sodium bicarbonate. The methylene chloride layer was then dried over anhydrous sodium carbonate, filtered, and stripped of volatiles. The residue was a sticky solid. It's identity was confirmed by NMR which showed the following peaks: 8.66 ppm (doublet, indicating 3'-proton); 8.44 ppm (doublet, indicating 5'-proton); 7.32 ppm (singlet, indicating 3,5-protons); 3.94 ppm (quartet, indicating α-protons); and 1.20 ppm (triplet, indicating the β-protons).

EXAMPLE 9: N-METHYL-2,3,4,5,6-PENTACHLORO-4'-NITRO-6'-(TRIFLUOROMETHYL)DIPHENYLAMINE

Sodium hydride (5.0 grams of a 57% oil dispersion) and 50 ml. of DMF (dried over molecular sieves) were combined and the resulting suspension was cooled to, and henceforth maintained at, between -10° and 0°C.

Nitrogen was passed over the reaction mixture throughout the reaction. A solution of N-methyl-2,3,4,5,6-pentachloroaniline (10.0 grams; 0.036 mole) in 50 ml. of DMF was added over a 10-minute period. The reaction mixture was allowed to stir for one hour. Then a solution of 2-chloro-5-nitrobenzotrifluoride (8.1

grams; 0.036 mole) in 50 ml. of DMF was added over a 15-minute period, and the reaction mixture was allowed to stir at room temperature for six hours.

The reaction mixture was then poured over ice and the volume adjusted to 1.8 l. with water. An oil formed; it was extracted with ether, dried, and the ether stripped off. The residual oil was recrystallized from hexane, m.p., 152-154°C. Its identity was confirmed by NMR and elemental analysis, which showed the following

Calculated for $C_{14}H_6Cl_5F_3N_2O_2$: C, 35.89; H, 1.29; N, 5.98.

Found: C, 36.16; H, 1.35; N, 6.02.

EXAMPLE 10: N-METHYL-2,3,4,5,6-PENTACHLORO-2',4'-DINITRO-6'-(TRIFLUOROMETHYL)DIPHENYLAMINE

N-Methyl-2,3,4,5,6-pentachloro-4'-nitro-6'-(trifluoromethyl)diphenylamine (1.85 grams; 0.0039 mole) was suspended in 40 ml. of trifluoroacetic acid. To this suspension there was added, by means of a dropping funnel, 0.37 gram (0.0044 mole) of ammonium nitrate in 12 ml. of trifluoroacetic acid. The reaction mixture was allowed to heat at 45-50°C. for 2 hours, then 100 ml. of water was added, and the reaction mixture was allowed to stir overnight (about 20 hours) at room temperature. The water was decanted off, leaving an oily residue which was recrystallized from ethanol, m.p., 159-161°C. Its identity was confirmed by NMR and elemental analysis, which showed

Calculated for $C_{14}H_5Cl_5F_3N_3O_4$: C, 32.75; H, 0.98; N, 8.18.

Found: C, 33.07; H, 1.21; N, 8.25.

The yield was 0.74 gram (37.0%).

EXAMPLE 11: N-METHYL-2,4-DIBROMO-2',4'-DINITRO-6'-(TRIFLUOROMETHYL)DIPHENYLAMINE

2,4-Dinitro-6-(trifluoromethyl)diphenylamine (11 grams; 0.034 mole), sodium carbonate (14 grams), dimethyl sulfate (6 ml.), and dioxane (to a total volume of 45 ml.), were stirred and refluxed for 24 hours. TLC indicated that the reaction was about 60% complete. Additional dimethyl sulfate (12 mls.) and sodium carbonate (10 grams) were added and the reaction continued for another two hours. The reaction mixture was then poured into water and stirred for four (4) hours. The supernatant was decanted and the residue was taken up in methylene chloride, separated from the water and filtered. To the resulting solution, containing 2,4-dinitro-6-(trifluoromethyl)-N-methyldiphenylamine, excess bromine was added and the reaction mixture was allowed to stand one hour, washed with water and sodium bicarbonate solution, filtered, and evaporated to dryness. The resulting residue was recrystallized from ethanol, m.p., 110°C. The yield was 11 grams (64.8%). The identity of the product was confirmed by NMR and elemental analysis, which showed

Calculated for $C_{14}H_8Br_2F_3N_3O_4$: C, 33.70; H, 1.62; N, 8.42.

Found: C, 33.95; H, 1.86; N, 8.32.

EXAMPLE 12: N-*n*-PROPYL-4-FLUORO-2',4'-DINITRO-6'-(TRIFLUOROMETHYL)DIPHENYLAMINE

The title compound may be prepared using techniques similar to those described above.

The diphenylamine derivatives shown in the following table were similarly prepared.

| EXAMPLE NUMBER | R | $R^1_m$ and $R^2_n$ | MELTING POINT (°C) OR ELEMENTAL ANALYSIS | PERCENT YIELD |
|---|---|---|---|---|
| 13 | $CH_3$ | 3-F | Calc. for $C_{14}H_9F_4N_3O_4$: Found:<br>C,46.81;H,2.53;N,11.70.<br>C,47.09;H,2.80;N,11.90. | 57.6 |
| 14 | $CH_3$ | 2-F | 83-84° | 67.6 |
| 15 | $CH_3$ | 2,5-di F | 109-110° | 26.5 |
| 16 | $CH_3$ | 3,4-di F | 91-93° | 74.6 |
| 17 | $CH_3$ | 2,6-di F | 95° | 28.4 |
| 18 | $CH_3$ | 2,3,5,6-tetra F | 94-95° | 50.7 |
| 19 | $CH_3$ | penta F | 114.5-115° | 22.3 |
| 20 | $CH_3$ | 4-Cl | 156-157° | 65.8 |
| 21 | $CH_3$ | 3-Cl | Calc. for $C_{14}H_9ClF_3N_3O_4$: Found:<br>C,44.76;H,2.41;N,11.18.<br>C,44.71;H,2.21;N,11.18. | 70.3 |
| 22 | $CH_3$ | 2-Cl | 140-141° | 36.1 |
| 23 | $CH_3$ | 2,4,6-tri Cl | 120-121.5° | 34.6 |
| 24 | $n-C_3H_7$ | 2,4,6-tri Cl | Calc. for $C_{16}H_{11}Cl_3F_3N_3O_4$: Found:<br>C,40.66;H,2.35;Cl,22.5;N,8.89.<br>C,40.66;H,2.22;Cl,22.45;N,8.71. | -(not recorded) |

| EXAMPLE NUMBER | R | $R^1_m$ and $R^2_n$ | MELTING POINT (°C) OR ELEMENTAL ANALYSIS | PERCENT YIELD |
|---|---|---|---|---|
| 25 | $CH_3$ | 4-Br | 167-168° | 95.2 |
| 26 | $C_2H_5$ | 4-Br | Calc. for $C_{15}H_{11}BrF_3N_3O_4$: Found: C,41.50;H,2.55;N,9.68. C,41.27;H,2.63;N,9.45. | 8.6 |
| 27 | $CH_3$ | 3-Br | 87-89° | 39.0 |
| 28 | $C_2H_5$ | 2,4-di Br | 131-132°C. | 67.9 |
| 29 | $CH_3$ | 4-I | 163-164° | 65.2 |
| 30 | $CH_3$ | 4-$CF_3$ | 134-137° | 91.8 |
| 31 | $CH_3$ | 3-$CF_3$ | 108-109° | 48.9 |
| 32 | $CH_3$ | 2-Cl-5-$CF_3$ | Calc. for $C_{15}H_8ClF_6N_3O_4$: Found: C,40.61;H,1.82;N,9.47. C,40.84;H,1.72;N,9.45. | 12.4 |
| 33 | $CH_3$ | 4-CN | 146-149° | 86.6 |
| 34 | $C_2H_5$ | 4-CN | 53-55° | 8.3 |
| 35 | $C_2H_5$ | 2,6-di Cl-4-CN | 118-120° | 21.0 |
| 36 | $C_2H_5$ | 2,4-di F | 75-76° | 3.6 |
| 37 | $CH_3$ | 3,5-di Cl | 107.5-108° | 73.2 |
| 38 | $CH_3$ | 2,4,6-tri Cl-3-F | 137-139° | 13.6 |
| 39 | $CH_3$ | 2,4-di Br-6-Cl | 139-141° | 78.7 |

| EXAMPLE NUMBER | R | $R^1_m$ and $R^2_n$ | MELTING POINT(°C) OR ELEMENTAL ANALYSIS | PERCENT YIELD |
|---|---|---|---|---|
| 40 | $CH_3$ | 3-CN | 109.5-110° | 56.8 |
| 41 | $CH_3$ | 2,6-di Br-4-F | 161.5-163° | 50.4 |
| 42 | $CH_3$ | 2-$NO_2$-4-Cl | 177.5-178.5° | 49.2 |
| 43 | $CH_3$ | 2-$CF_3$ | 148-149° | 3.7 |
| 44 | $CH_3$ | 2,6-di Cl-4-$CF_3$ | 98.5-99° | 68.5 |
| 45 | $CH_3$ | 2,4-di F-6-$NO_2$ | 102-103° | 46.1 |
| 46 | $CH_3$ | 2-Br-4-Cl | 131-133° | 23.8 |
| 47 | $CH_3$ | 2,6-di Br-4-Cl | 164.5-165.5° | 69.1 |
| 48 | $CH_3$ | 2,6-di Cl-4-F | 132-134° | 38.6 |
| 49 | $CH_3$ | 3-$NO_2$ | 112-113° | 65.2 |
| 50 | $CH_3$ | 3,4-di Cl | 107-109° | 49.4 |
| 51 | $CH_3$ | 2,4-di Cl | 97-99° | 14.4 |
| 52 | $CH_3$ | 3,4-di Br | 151-152° | 68.1 |
| 53 | $CH_3$ | 2-Br-4-F-6-Cl | 151-152° | 68.5 |
| 54 | $CH_3$ | 2,4-di F-6-Br | 144-145° | 48.8 |
| 55 | $CH_3$ | 2,4-di F-6-Cl | 123-124° | 57.0 |
| 56 | $CH_3$ | 2-Cl-4-F | 130-131° | 71.5 |

EXAMPLE 57: MITE-INSECT SCREEN

Numerous of the compounds to be employed in the present invention were evaluated against four insect and mite species, with test procedures as set forth below.

Mexican Bean Beetle & Southern Armyworm

Bountiful green beans were grown from seed to 4- to 6-day-old plants. They were then sprayed with a formulation containing the test compound at 1000 ppm or lesser concentration; the spraying was of both the tops and bottoms of the leaves, about 8-10 ml. per plant. The plants were then allowed to dry and the leaves removed.

Some of the leaves were placed in 100 x 20 mm plastic petri dishes containing second instar larvae of Mexican Bean Beetle (Epilachna varivestis Mulsant, order Coleoptera, family Coccinellidae), two leaves (approximately 6 sq. in. of leaf surface) and five larvae per petri dish. Two replicates were carried out per treatment. A small pad of cellulose was added to each dish to help prevent the leaves from wilting.

Other treated leaves were placed in 100 x 20 mm plastic petri dishes containing third instar larvae of Southern Armyworm (Spodoptera eridania Cramer, order Lepidoptera, Family Noctuidae).

A solvent control and an untreated control were also employed, as well as a reference standard for each species.

For both species, mortality counts were made four days after the larvae were placed on the treated leaves, using the following scale:

| Actual No. of dead larvae | Rating |
|---|---|
| 0 | 0 |
| 1-2 | 1 |
| 3-4 | 2 |
| 5 | 3 |

In some tests, the amount of feeding by the larvae was also rated, on the following scale:

| Feeding | Rating |
|---|---|
| none | 0 |
| 1-50% of leaves | 1 |
| 50-99% of leaves | 2 |
| 100% of leaves | 3 |

Two-spotted Spider Mite

Young Blue hubbard squash plants were thinned to one cotyledon per plant and infested by placing on each cotyledon a bean leaf infested with two-spotted spider mite (*Tetranychus urticae* (Koch), Order Acarina, family Tetranychidae). The plants were held for a day, then sprayed to wetting with a formulation containing 1000 ppm or lesser concentration of the test compound, with one infested plant (cotyledon) per treatment. Mortality was determined four days after treatment, using the following rating scale:

| Percent Dead | Rating |
|---|---|
| 0 | 0 |
| 1-50 | 1 |
| 51-99 | 2 |
| 100 | 3 |

## Housefly

A cage of about 100 houseflies (*Musca domestica* (Linne), Order Diptera, Family Muscidae) was sprayed with 5 ml. of a formulation containing 1000 ppm or lesser concentration of the test compound. A knockdown count was made 2 hours later, after which the flies were supplied a 5% solution of sugar in water. A mortality count was made 24 hours after treatment. Both knockdown and mortality counts employed the same rating system employed in the two-spotted spider mite test. An untreated control, a solvent control, and a reference standard were employed.

Results of the foregoing tests were as set forth in the following table. For the Mexican Bean Beetle and Southern Armyworm species, each of the two replicates is reported separately.

TABLE II: MITE-INSECT SCREEN

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 2 | 1000 | 3,3 | 0,0 | 0,0 | | 3 | | |
| | 1000 | 3,2 | 1,1 | 0,0 | | 3 | | |
| | 100 | 0,0 | | | | 0 | | |
| | 1000 | 3,3 | 1,1 | | | 0 | | |
| 3 | 1000 | 3,3 | 0,0 | 3,3 | 1,0 | 3 | | |
| | 10 | 1,0 | | 0,0 | | 0 | | |
| | 100 | 3,3 | 1,1 | 1,0 | | 3 | 3 | 2 |
| | 1000 | 3,3 | 0,0 | 3,3 | 0,0 | 3 | 3 | 3 |
| | 10 | 0,0 | | 0,0 | | 0 | | |
| | 100 | 0,0 | | 0,0 | | 0 | | |
| 4 | 1000 | 3,3 | 0,0 | 3,3 | 0,0 | 3 | | |
| | 1000 | 3,3 | 0,0 | 3,3 | 0,0 | 0 | | |
| | 100 | 1,1 | | 3,3 | 0,0 | | | |
| | 10 | | | 0,0 | | | | |
| | 100 | 0,0 | | 3,3 | 0,0 | 3 | | |
| | 1000 | 3,3 | 0,0 | 3,3 | 0,0 | 3 | | |
| 5 | 100 | 1,0 | | 0,0 | | | | |
| | 1000 | 3,3 | 0,1 | 3,3 | 0,0 | 0 | | |
| | 10 | 0,0 | | 0,0 | | 3 | | |
| | 100 | 2,2 | 1,1 | 1,0 | | 3 | | |
| | 1000 | 3,3 | 0,0 | 3,3 | 0,0 | 2 | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 6 | 10 | 1,0 | | 0,0 | | 0 | | |
| | 100 | 0,0 | | 0,0 | | 0 | | |
| | 1000 | 0,0 | | 3,3 | 0,0 | 1 | | |
| 10 | 1000 | 3,3 | 0,0 | 3,3 | 1,1 | 0 | | |
| | 1000 | 3,3 | 1,1 | 3,2 | 1,1 | 2 | | |
| | 100 | 0,0 | | | | | | |
| 11 | 1000 | 3,3 | 1,1 | 3,3 | 0,1 | 3 | | |
| | 1000 | 3,3 | 1,1 | 3,3 | 0,0 | 2 | | |
| | 100 | 3,1 | 1 | 3,3 | 1,0 | 1,1 | | |
| | 1000 | 3,3 | 1,0 | 3,3 | 0,0 | 3,3 | 3 | 0 |
| | 10 | 0,0 | | 0,0 | | | 2 | 2 |
| | 100 | 2,3 | | 3,3 | | | 2 | 2 |
| | 100 | 3,3 | 0,1 | | | | | |
| | 10 | 0,0 | | | | | | |
| 13 | 1000 | 2,3 | 1,1 | 0,0 | | 3 | | |
| 14 | 1000 | 3,3 | 0,0 | 2,1 | 1 | 0 | | |
| | 1000 | 3,3 | 0,0 | 2,3 | 0 | 2 | | |
| | 100 | 0,0 | | | | | | |
| | 1000 | 3,3 | 1,1 | | | | | |
| | 100 | 1,0 | | 0,0 | | | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 15 | 1000 | 3,3 | 0,1 | 3,3 | 0,0 | 0 | | |
| | 1000 | 3,3 | 0,0 | 3,3 | 0,0 | 3 | | |
| | 10 | 0,0 | | 0,0 | | 0 | | |
| | 100 | 1,0 | | 1,1 | 1,1 | 0 | 2 | 1 |
| | 1000 | 3,3 | 0,1 | 3,3 | 0,0 | 0 | 3 | 3 |
| | 100 | 1,1 | | 1,3 | 0 | | | |
| 16 | 1000 | 3,3 | 0,0 | 2,3 | 0,0 | 3 | | |
| | 1000 | 3,3 | 1,1 | 3,3 | 0,0 | 3 | | |
| | 100 | 1,1 | | | | | | |
| | 1000 | 3,3 | 0,1 | | | | | |
| | 100 | 0,0 | | 2,0 | | | | |
| 17 | 1000 | 3,3 | 0,0 | 3,3 | 0,0 | 3 | | |
| | 100 | 1,0 | | 2,0 | | | | |
| 18 | 1000 | 3,3 | 0,0 | 3,3 | 0,0 | 2 | | |
| | 1000 | 3,3 | 1,0 | 3,3 | 0,0 | 3 | | |
| | 100 | 0,0 | | 2,3 | 1,0 | | | |
| | 100 | 1,1 | | 3,3 | 0,0 | 0 | 0 | |
| | 1000 | 3,3 | 0,0 | 3,3 | 0,0 | 2 | 3 | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 19 | 1000 | 1,1 | | 3,3 | 0,0 | 3 | | |
| | 100 | | | 3,3 | 0,0 | | | |
| | 10 | | | 0,0 | | | | |
| 20 | 1000 | 2,3 | | 1,1 | | 2 | | |
| | | 0,0 | | 0,0 | | 0 | | |
| 21 | 1000 | 3,3 | 1,1 | 2,2 | | 3 | | |
| | 100 | 0,0 | | | | | | |
| 22 | 1000 | 3,3 | 1,1 | 1,1 | 1 | 1 | | |
| | 100 | 1,1 | | | | | | |
| 23 | 1000 | 3,3 | 0,0 | 3,3 | 0,0 | 3,3 | 3 | 3 |
| | 1000 | 2,2 | 1,1 | 3,2 | 0,0 | 1,2 | | |
| | 1000 | 3,3 | 0,0 | 3,3 | 0,0 | 0 | | |
| | 1000 | 3,3 | 1,1 | 3,3 | 0,0 | 3 | | |
| | 100 | 2,1 | | 3,3 | 0,1 | 1,1 | 2 | 0 |
| | 10 | 0,0 | | 0,0 | | 0,0 | | |
| | 100 | 0,1 | | 3,3 | 0,0 | | | |
| | 10 | | | 0,0 | | | | |
| 25 | 1000 | 0,0 | | 0,1 | | 3 | | |
| | 1000 | 1,1 | | 1,1 | 1 | 3 | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 27 | 1000 | 3,3 | 1,1 | 3,2 | 1,1 | 3 | | |
| | 100 | 0,0 | | 0,0 | | | | |
| 28 | 1000 | 3,3 | 1,0 | 3,3 | 0,1 | 3 | | |
| | 100 | 3,3 | 1,0 | 3,3 | | | 3 | 2 |
| | 1000 | 3,3 | | 3,3 | 0,0 | | 3 | 3 |
| | 10 | 0 | 0 | 0 | 0 | | 0 | 0 |
| | 100 | 1 | 1 | 3 | 3 | | 3 | 3 |
| 29 | 1000 | 1,1 | | 2,2 | | 3 | | |
| 30 | 1000 | 3,3 | 1,0 | 1,1 | | 3 | | |
| | 1000 | 3,3 | | 2,3 | 1,0 | 3 | | |
| | 100 | 2,2 | 1,1 | 0,0 | | 0 | | |
| | 1000 | 3 | 0 | 3 | 0 | 3 | | |
| | 100 | 1,0 | | | | | | |
| 31 | 1000 | 3,3 | 0,0 | 3,3 | 0,0 | 3 | | |
| | 100 | 1,0 | | 0,0 | | | | |
| 32 | 1000 | 1,0 | | 3,3 | 0,0 | 3 | | |
| | 100 | | | 0,1 | | | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 33 | 1000 | 2,3 | 1,1 | 0,0 | | 0 | | |
| | 1000 | 1,1 | | 0,0 | | 3 | | |
| | 100 | 0,0 | | | | | | |
| | 1000 | 3 | 1 | | | | | |
| 35 | 1000 | 3,3 | 1,1 | 0,0 | | 0 | | |
| | 100 | 0,0 | | | | | | |
| 37 | 1000 | 1,1 | | 3,3 | 1,0 | 0 | | |
| | 1000 | 0,0 | | 3,3 | 0,0 | 0 | | |
| | 10 | 0,0 | | 0,0 | 0,0 | 0 | 2 | 2 |
| | 100 | 0,2 | 1 | 3,2 | 0,0 | 2 | 3 | 3 |
| | 1000 | 3,3 | 1 | 3,3 | | | | |
| | 100 | | | 3,2 | 0,0 | | | |
| | 1000 | | | 3,3 | 0,0 | | | |
| 38 | 1000 | 3,3 | 1,1 | 3,2 | 0,1 | 2 | | |
| | 100 | 0,0 | | 0,0 | | | | |
| 39 | 1000 | 3,3 | 1,0 | 3,3 | 1,0 | 0 | | |
| | 1000 | 0,3 | 1 | 3,3 | 0,0 | | | |
| | 1000 | 3,3 | 0,0 | 3,3 | 0,0 | 3 | | |
| | 100 | 0,0 | | 0,0 | | | | |
| | 100 | 0,0 | | 1,0 | | | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 40 | 1000 | 3,3 | 1,0 | 0,1 | | 3 | | |
| | 1000 | 3,3 | 1,1 | 0,0 | | 2 | | |
| | 100 | 0,0 | | | | | | |
| 41 | 1000 | 3,2 | 1,1 | 3,3 | 0,1 | 2 | | |
| | 100 | 0,0 | | 0,0 | | | | |
| | 1000 | 0,0 | | 3,3 | 0,0 | 0 | | |
| 42 | 1000 | 0,0 | | 3,3 | 1,1 | 1 | | |
| | 100 | | | 1,2 | | | | |
| 43 | 1000 | 0,0 | 1 | 1,1 | | 1 | | |
| 45 | 1000 | 3,3 | 0,0 | 3,3 | 0,0 | 3 | | |
| | 100 | 1,0 | | 1,0 | | 0 | 3 | 0 |
| | 1000 | 3,3 | 1,1 | 3,3 | 0,0 | 0 | 3 | 2 |
| | 10 | | | | | 0 | 0 | 0 |
| | 100 | 0 | 0 | 0 | 0 | 0 | 3 | 1 |
| | 100 | 0,0 | | 0,0 | | 0 | | |
| | 1000 | 3,3 | 0,1 | 3,3 | 1,1 | 1 | | |
| | 10 | 1,0 | | 0,0 | | 0 | | |
| | 100 | 0,1 | | 0,0 | | 0 | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| | 10 | 1,1 | | 0,0 | | 0 | | |
| | 100 | 0,0 | | 1,0 | | 0 | | |
| | 1000 | 3,3 | 1,1 | 3,3 | 0,0 | 3 | | |
| 45 | 1000 | 2,2 | | 3,3 | 0,0 | 3 | | |
| | 10 | 0,0 | | 0,0 | | 0 | | |
| | 100 | 0,0 | | 2,2 | 1,1 | 3 | | |
| | 1000 | 1,1 | 1,1 | 3,3 | 0,0 | 3 | | |
| 47 | 1000 | 0,0 | | 3,3 | 0,0 | 3 | | |
| | 10 | 0,0 | | 0,0 | | 3 | | |
| | 100 | 0,0 | | 0,0 | | 1 | | |
| | 1000 | 0,0 | | 3,3 | 0,0 | 1 | | |
| | 100 | 0,0 | | 2,2 | | 0 | | |
| | 1000 | 1,0 | | 3,3 | | 1 | | |
| 48 | 1000 | 1,0 | | 3,3 | 0,0 | 0 | | |
| | 10 | 0,0 | | 0,0 | | 1 | | |
| | 100 | 2,1 | 1,1 | 2,2 | 0,1 | 1 | | |
| | 1000 | 3,3 | 0,0 | 3,3 | 0,0 | 3 | | |
| 49 | 10 | 0,0 | | 0,0 | | 1 | | |
| | 100 | 1,0 | | 0,0 | | 2 | | |
| | 1000 | 3,3 | 0,0 | 3,3 | 0,0 | 3 | | |

TABLE II (cont'd.)

| Compound of Example No. | Appln. Rate ppm. | Mexican Bean Beetle Mortality Rating | Mexican Bean Beetle Feeding Rating | Southern Armyworm Mortality Rating | Southern Armyworm Feeding Rating | Two-spotted Spider Mite Mortality Rating | Housefly Mortality Rating Contact | Housefly Mortality Rating Knockdown |
|---|---|---|---|---|---|---|---|---|
| 50 | 1000 | 3,3 | | 3,3 | 0,0 | 3 | | |
| | 1000 | 3,3 | 0,0 | 3,3 | 0,0 | 3 | | |
| | 10 | 1,1 | | 0,0 | | 0 | | |
| | 100 | 3,3 | | 3,3 | 0,0 | 3 | | |
| 51 | 1000 | 3,3 | 0,0 | 3,3 | 0,0 | 3 | | |
| | 10 | 2,1 | | 0,0 | | 2 | | |
| | 100 | 3,3 | 1,1 | 3,3 | 0,0 | 3 | | |
| 52 | 1000 | 3,3 | 0,0 | 3,3 | 0,0 | 3 | | |
| | 10 | 0,0 | | 0,0 | | 0 | | |
| | 100 | 0,0 | | 3,3 | 0,0 | 1 | | |
| 53 | 1000 | 3,2 | 1,1 | 3,3 | 0,0 | 3 | | |
| | 10 | 0,0 | | 0,0 | | 1 | | |
| | 100 | 0,0 | | 2,2 | 1,1 | 2 | | |
| 54 | 1000 | 3,3 | 0,1 | 3,3 | 0,0 | 3 | | |
| | 10 | 0,0 | | 0,0 | | 2 | | |
| | 100 | 0,0 | | 3,3 | 0,0 | 3 | | |
| 55 | 1000 | 3,3 | 1,0 | 3,3 | 0,0 | 3 | | |
| | 10 | 0,0 | | 0,0 | | 2 | | |
| | 100 | 0,0 | | 3,3 | 0,0 | 3 | | |
| 56 | 1000 | 3,3 | 0,0 | 3,3 | 0,0 | 3 | | |
| | 10 | 0,0 | | 0,0 | | 1 | | |
| | 100 | 3,3 | 1,1 | 2,3 | 1,0 | 3 | | |

EXAMPLE 58: TWO-SPOTTED SPIDER MITE TEST

Numerous of the compounds to be employed in accordance with the present invention were evaluated for the control of the two-spotted spider mite (Tetranychus urtiçae) on plants of dry bean (variety Kentucky Wonder).

Each compound to be tested was formulated by dissolving it in a 1:1 mixture of acetone and anhydrous ethyl alcohol containing 23 grams of Toximul R and 13 grams of Toximul S per liter of solvent.

The testing procedure was as follows. Ten-day-old bean plants were trimmed until one stem with one leaf remained per plant, mite-infested leaves were placed on the trimmed plants and the mites were allowed to transfer for one to three days (generally two days). The infesting leaves were then removed and the newly infested plants were sprayed to the point of runoff with one of the test formulations and then maintained under favorable conditions. One to three days later (generally two days later) the degree of miticidal action was determined by counting the number of mites surviving in a total of eight microscope fields, about 6.28 sq. cm. of leaf area. Population counting was stopped as soon as 25 mites had been counted. Four replicates were conducted.

The following table records the results as the mean of the four replicates. Not all of the compounds were evaluated at the same time, but a solvent control (solvent concentration ranging from 4,000 ppm to 100,000 ppm) was included with each set of evaluations. Except where otherwise indicated, the mean solvent control number of mites was in each instance in excess of 25.

Plant injury ratings were generally made, on the following scale:

0 = no injury
1 = slight injury
2 = moderate injury
3 = severe injury

Results are reported in the following table.

TABLE III:

TWO-SPOTTED SPIDER MITE TEST

| Compound of Example No. | Dosage ppm. | Mean Number of Mites per 6.28 sq. cm. | Plant Injury Rating |
|---|---|---|---|
| 2 | 1000 | 2/ 0.00 | 0 |
| | 100 | 0.00 | 0 |
| | 50 | 0.75 | 0 |
| 2 | 100 | 0.50 | 0 |
| | 50 | 4.50 | 0 |
| | 25 | 22.75 | 0 |
| | 10 | 25.00 | 0 |
| 2 | 100 | 0.00 | 0 |
| | 50 | 1.50 | 0 |
| | 25 | 5.25 | 0 |
| | 10 | 14.50 | 0 |
| 2 | 1000 | 0.00 | - |
| | 100 | 1.00 | - |
| | 10 | 24.50 | - |
| 2 | 100 | 2.25 | 0 |
| | 50 | 5.50 | 0 |
| | 10 | 11.75 | 0 |

TABLE III (cont'd.)

| Compound of Example No. | Dosage ppm. | Mean Number of Mites per 6.28 sq. cm. | Plant Injury Rating |
|---|---|---|---|
| 2 | 100 | 5/ 1.25 | 0 |
| | 50 | 0.75 | 0 |
| | 10 | 18.25 | 0 |
| 3 | 1000 | 0.00 | 0 |
| | 100 | 0.00 | 0 |
| | 50 | 0.25 | 0 |
| 3 | 100 | 0.00 | 0 |
| | 50 | 0.25 | 0 |
| | 25 | 2.25 | 0 |
| | 10 | 9.00 | 0 |
| 3 | 100 | 1.25 | 0 |
| | 50 | 5.50 | 0 |
| | 25 | 17.50 | 0 |
| | 10 | 23.50 | 0 |
| 3 | 100 | 0.00 | 0 |
| | 50 | 1.50 | 0 |
| | 25 | 12.50 | 0 |
| | 10 | 25.00 | 0 |
| 3 | 100 | 0.00 | 0 |
| | 50 | 1.00 | 0 |
| | 25 | 1.75 | 0 |
| | 10 | 3.75 | 0 |
| 3 | 1000 | 1/ 0.00 | 0 |
| | 100 | 0.00 | 0 |
| | 50 | 0.00 | 0 |
| 3 | 100 | 3.00 | 0 |
| | 50 | 2.00 | 0 |
| | 25 | 14.50 | 0 |
| | 10 | 23.50 | 0 |

TABLE III (cont'd.)

| Compound of Example No. | Dosage ppm. | Mean Number of Mites per 6.28 sq. cm. | Plant Injury Rating |
|---|---|---|---|
| 3 | 1000 | 0.00 | 0 |
| | 100 | 1.50 | 0 |
| | 50 | 10.25 | 0 |
| | 25 | 10.75 | 0 |
| | 10 | 25.00 | 0 |
| 3 | 1000 | 0.00 | - |
| | 100 | 0.00 | - |
| | 10 | 8.00 | - |
| 3 | 100 | 0.00 | 0 |
| | 50 | 0.00 | 0 |
| | 10 | 3.50 | 0 |
| 3 | 100 | 5/ 0.25 | 0 |
| | 50 | 0.25 | 0 |
| | 10 | 8.25 | 0 |
| 3 | | nymphal and adult / larval | |
| | 1000 | 0.00 / 0.50 | 0 |
| | 100 | 3.00 / 13.75 | 0 |
| | 50 | 2.00 / 8.50 | 0 |
| | 10 | 18.50 / 11.75 | 0 |
| 3 | 100 | 6/(18 hrs.) 0.00 | - |
| | 50 | 0.25 | - |
| | 10 | 14.00 | - |
| 3 | 100 | 6/(24 hrs.) 0.50 | - |
| | 50 | 0.50 | - |
| | 10 | 23.25 | - |
| 3 | 100 | 6/(42 hrs.) 0.25 | - |
| | 50 | 5.25 | - |
| | 10 | 25.00 | - |

TABLE III (cont'd.)

| Compound of Example No. | Dosage ppm. | Mean Number of Mites per 6.28 sq. cm. | | Plant Injury Rating |
|---|---|---|---|---|
| 3 | 100 | 6/(48 hrs.) 2.00 | | - |
| | 50 | 14.75 | | - |
| | 10 | 25.00 | | - |
| 3 | 100 | 6/(66 hrs.) 6.50 | | - |
| | 50 | 14.00 | | - |
| | 10 | 25.00 | | - |
| 3 | 100 | 6/(72 25.00 | | - |
| | 50 | 16.00 | | - |
| | 10 | 25.00 | | - |
| 3 | 100 | 6/(90 hrs.) 20.25 | | - |
| | 50 | 25.00 | | - |
| | 10 | 25.00 | | - |
| 3 | | treatment one day after infestation | treatment three days after infestation | |
| | 100 | 0.50 | 0.25 | - |
| | 50 | 4.25 | 3.50 | - |
| | 25 | 12.00 | 10.75 | - |
| | 10 | 21.25 | 17.50 | - |
| 3 | 100 | 1.25 | | 0 |
| | 50 | 18.50 | | 0 |
| | 25 | 24.75 | | 0 |
| | 10 | 25.00 | | 0 |
| | 5 | 25.00 | | 0 |
| | 1 | 25.00 | | 0 |
| 4 | 1000 | 0.00 | | 0 |
| | 100 | 0.00 | | 0 |
| | 50 | 0.00 | | 0 |

TABLE III (cont'd.)

| Compound of Example No. | Dosage ppm. | Mean Number of Mites per 6.28 sq. cm. | Plant Injury Rating |
|---|---|---|---|
| 4 | 100 | 0.00 | 0 |
| | 50 | 0.25 | 0 |
| | 25 | 0.00 | 0 |
| | 10 | 2.00 | 0 |
| 5 | 100 | 0.00 | 0 |
| | 50 | 0.50 | 0 |
| | 25 | 1.00 | 0 |
| | 10 | 11.25 | 0 |
| 5 | 50 | 2.25 | 0 |
| | 25 | 10.00 | 0 |
| | 10 | 25.00 | 0 |
| 5 | 1000 | 0.00 | - |
| | 100 | 0.00 | - |
| | 10 | 2.75 | - |
| 5 | 1000 | 3/ 0.00 | 0 |
| | 100 | 0.25 | 0 |
| | 50 | 0.00 | 0 |
| 5 | 100 | 0.00 | 0 |
| | 50 | 0.25 | 0 |
| | 25 | 2.25 | 0 |
| | 10 | 0.50 | 0 |
| 5 | 100 | 0.25 | 0 |
| | 50 | 5.25 | 0 |
| | 25 | 4.25 | 0 |
| | 10 | 6.50 | 0 |

TABLE III (cont'd.)

| Compound of Example No. | Dosage ppm. | Mean Number of Mites per 6.28 sq. cm. (nymphal and adult) | | Mean Number of Mites per 6.28 sq. cm. (larval) | Plant Injury Rating |
|---|---|---|---|---|---|
| 5 | 100 | 0.00 | | 0.00 | 0 |
| | 50 | 0.00 | | 1.50 | 0 |
| | 25 | 2.00 | | 10.50 | 0 |
| | 10 | 3.75 | | 13.50 | 0 |
| | 5 | 25.00 | | 25.00 | 0 |
| | 1 | 25.00 | | 25.00 | 0 |
| 5 | 1000 | 0.00 | | 0.00 | 0 |
| | 100 | 0.00 | | 0.00 | 0 |
| | 50 | 0.00 | | 0.75 | 0 |
| | 10 | 1.25 | | 7.00 | 0 |
| 5 | 100 | 6/(18 hrs.) | 0.00 | | - |
| | 50 | | 0.25 | | - |
| | 10 | | 14.00 | | - |
| 5 | 100 | 6/(24 hrs.) | 0.25 | | - |
| | 50 | | 0.00 | | - |
| | 10 | | 7.75 | | - |
| 5 | 100 | 6/(42 hrs.) | 0.00 | | - |
| | 50 | | 0.00 | | - |
| | 10 | | 15.50 | | - |
| 5 | 100 | 6/(48 hrs.) | 0.25 | | - |
| | 50 | | 0.75 | | - |
| | 10 | | 13.25 | | - |

TABLE III (cont'd.)

| Compound of Example No. | Dosage ppm. | Mean Number of Mites per 6.28 sq. cm. | | Plant Injury Rating |
|---|---|---|---|---|
| 5 | 100 | 6/(66 hrs.) | 6.25 | 0 |
| | 50 | | 5.75 | 0 |
| | 10 | | 2.25 | 0 |
| 5 | 100 | 6/(72 hrs.) | 6.75 | - |
| | 50 | | 10.50 | - |
| | 10 | | 12.25 | - |
| 5 | 100 | 6/(90 hrs.) | 10.25 | - |
| | 50 | | 20.00 | - |
| | 10 | | 16.00 | - |
| 5 | | treatment one day after infestation | treatment three days after infestation | |
| | 50 | 3.75 | 0.25 | - |
| | 25 | 4.50 | 1.75 | - |
| | 10 | 22.50 | 2.25 | - |
| 6 | 4000 | | 1.50 | 0 |
| | 2000 | | 2.00 | 0 |
| | 1000 | | 14.50 | 0 |
| | 100 | | 18.50 | 0 |
| | 50 | | 21.00 | 0 |
| 6 | 4000 | | 5.25 | 0 |
| | 2000 | | 5.25 | 0 |
| | 1000 | | 3.25 | 0 |
| | 100 | | 13.00 | 0 |
| | 50 | | 20.25 | 0 |
| 8 | 1000 | | 0.00 | 0 |
| | 100 | | 0.25 | 0 |
| | 50 | | 0.50 | 0 |

TABLE III (cont'd.)

| Compound of Example No. | Dosage ppm. | Mean Number of Mites per 6.28 sq. cm. | Plant Injury Rating |
|---|---|---|---|
| 10 | 1000 | 1/ 4.25 | 0 |
| | 100 | 25.00 | 0 |
| | 50 | 25.00 | 0 |
| 11 | 100 | 0.75 | 0 |
| | 50 | 3.25 | 0 |
| | 25 | 5.25 | 0 |
| | 10 | 14.00 | 0 |
| 12 | 1000 | 14.50 | 0 |
| | 100 | 25.00 | 0 |
| | 50 | 25.00 | 0 |
| | 25 | 25.00 | 0 |
| | 10 | 25.00 | 0 |
| 13 | 1000 | 0.00 | 0 |
| | 100 | 25.00 | 0 |
| | 50 | 25.00 | 0 |
| 14 | 1000 | 2/ 15.75 | 0 |
| | 100 | 25.00 | 0 |
| | 50 | 25.00 | 0 |
| 15 | 1000 | 13.50 | 0 |
| | 100 | 23.75 | 0 |
| | 50 | 25.00 | 0 |
| 15 | 4000 | 14.25 | 0 |
| | 2000 | 24.75 | 0 |
| | 1000 | 22.75 | 0 |
| 16 | 1000 | 0.00 | 0 |
| | 100 | 0.00 | 0 |
| | 50 | 1.75 | 0 |
| 16 | 100 | 0.00 | 0 |
| | 50 | 3.00 | 0 |
| | 25 | 5.50 | 0 |
| | 10 | 8.00 | 0 |

TABLE III (cont'd.)

| Compound of Example No. | Dosage ppm. | Mean Number of Mites per 6.28 sq. cm. | Plant Injury Rating |
|---|---|---|---|
| 17 | 1000 | 0.50 | 0 |
| | 100 | 25.00 | 0 |
| | 50 | 18.75 | 0 |
| 18 | 1000 | 1/ 0.50 | 0 |
| | 100 | 18.00 | 0 |
| | 50 | 23.00 | 0 |
| 19 | 1000 | 2.00 | 0 |
| | 100 | 15.25 | 0 |
| | 50 | 23.50 | 0 |
| 20 | 100 | 0.00 | 0 |
| | 50 | 0.00 | 0 |
| | 25 | 4.25 | 0 |
| | 10 | 9.25 | 0 |
| 20 | 100 | 10.25 | 0 |
| | 50 | 3.00 | 0 |
| | 25 | 22.00 | 0 |
| | 10 | 19.50 | 0 |
| 20 | 1000 | 4.00 | 0 |
| | 100 | 0.00 | 0 |
| | 50 | 0.25 | 0 |
| 20 | 1000 | 2.00 | 0 |
| | 100 | 7.25 | 0 |
| | 50 | 23.50 | 0 |
| 20 | 1000 | 1.75 | 0 |
| | 100 | 0.50 | 0 |
| | 50 | 1.25 | 0 |
| 20 | 1000 | 0.50 | - |
| | 100 | 0.00 | - |
| | 10 | 18.00 | - |

TABLE III (cont'd.)

| Compound of Example No. | Dosage ppm. | Mean Number of Mites per 6.28 sq. cm. | Plant Injury Rating |
|---|---|---|---|
| 20 | 100 | 1.25 | 0 |
| | 50 | 0.25 | 0 |
| | 10 | 4.50 | 0 |
| 20 | 100 | 5/ 0.00 | 0 |
| | 50 | 0.75 | 0 |
| | 10 | 1.00 | 0 |
| 21 | 1000 | 0.25 | 0 |
| | 100 | 1.75 | 0 |
| | 50 | 6.75 | 0 |
| 22 | 1000 | 18.50 | 0 |
| | 100 | 25.00 | 0 |
| | 50 | 25.00 | 0 |
| 23 | 1000 | 0.00 | 0 |
| | 100 | 1.00 | 0 |
| | 50 | 7.25 | 0 |
| 23 | 1000 | 0.00 | 0 |
| | 100 | 0.00 | 0 |
| | 50 | 0.00 | 0 |
| 23 | 100 | 2.25 | 0 |
| | 50 | 9.00 | 0 |
| | 25 | 5.00 | 0 |
| | 10 | 21.25 | 0 |
| 23 | 1000 | 0.00 | 0 |
| | 100 | 0.00 | 0 |
| | 50 | 0.00 | 0 |
| 23 | 1000 | 0.00 | 0 |
| | 100 | 1.00 | 0 |
| | 50 | 7.25 | 0 |
| 24 | 1000 | 0.00 | 0 |
| | 100 | 0.25 | 0 |
| | 50 | 0.00 | 0 |

TABLE III (cont'd.)

| Compound of Example No. | Dosage ppm. | Mean Number of Mites per 6.28 sq. cm. | Plant Injury Rating |
|---|---|---|---|
| 25 | 100 | 0.00 | 0 |
| | 50 | 1.00 | 0 |
| | 25 | 4.00 | 0 |
| | 10 | 6.50 | 0 |
| 25 | 1000 | 2.25 | 0 |
| | 100 | 7.00 | 0 |
| | 50 | 1.75 | 0 |
| 25 | 1000 | 0.00 | 0 |
| | 100 | 1.25 | 0 |
| | 50 | 2.50 | 0 |
| 25 | 100 | 10.75 | 0 |
| | 50 | 9.00 | 0 |
| | 25 | 6.25 | 0 |
| | 10 | 21.25 | 0 |
| 25 | 1000 | 0.75 | - |
| | 100 | 0.25 | - |
| | 10 | 20.25 | - |
| 25 | 100 | 0.75 | 0 |
| | 50 | 1.00 | 0 |
| | 10 | 14.00 | 0 |
| 25 | 100 | 5/ 0.00 | 0 |
| | 50 | 1.75 | 0 |
| | 10 | 15.25 | 0 |
| 26 | 1000 | 0.00 | 0 |
| | 100 | 4.50 | 0 |
| | 50 | 7.75 | 0 |
| 26 | 1000 | 0.00 | 0 |
| | 100 | 0.25 | 0 |
| | 50 | 1.75 | 0 |
| 27 | 1000 | 0.75 | 0 |
| | 100 | 17.00 | 0 |
| | 50 | 17.25 | 0 |

TABLE III (cont'd.)

| Compound of Example No. | Dosage ppm. | Mean Number of Mites per 6.28 sq. cm. | Plant Injury Rating |
|---|---|---|---|
| 28 | 2000 | 0.00 | - |
| | 500 | 0.25 | - |
| | 100 | 3.5 | - |
| | 50 | 11.25 | - |
| | 25 | 17.75 | - |
| 29 | 100 | 0.00 | 0 |
| | 50 | 0.25 | 0 |
| | 25 | 2.50 | 0 |
| | 10 | 8.25 | 0 |
| 29 | 1000 | 0.00 | 0 |
| | 100 | 0.25 | 0 |
| | 50 | 1.25 | 0 |
| 29 | 100 | 1.25 | 0 |
| | 50 | 2.25 | 0 |
| | 25 | 3.75 | 0 |
| | 10 | 17.75 | 0 |
| 30 | 4000 | 21.75 | 0 |
| | 2000 | 22.75 | 0 |
| | 1000 | 23.50 | 0 |
| 30 | 1000 | 0.25 | 0 |
| | 100 | 3.50 | 0 |
| | 50 | 7.50 | 0 |
| 31 | 100 | 0.00 | 0 |
| | 50 | 0.25 | 0 |
| | 25 | 1.25 | 0 |
| | 10 | 8.00 | 0 |
| 31 | 1000 | 0.00 | 0 |
| | 100 | 0.25 | 0 |
| | 50 | 0.25 | 0 |
| 31 | 1000 | 0.00 | 0 |
| | 100 | 0.00 | 0 |
| | 50 | 0.25 | 0 |

TABLE III (cont'd.)

| Compound of Example No. | Dosage ppm. | Mean Number of Mites per 6.28 sq. cm. | Plant Injury Rating |
|---|---|---|---|
| 31 | 1000 | 0.00 | - |
| | 100 | 0.50 | - |
| | 10 | 9.50 | - |
| 31 | 100 | 0.25 | 0 |
| | 50 | 1.25 | 0 |
| | 10 | 3.25 | 0 |
| 31 | 100 | 5/ 0.00 | 0 |
| | 50 | 0.25 | 0 |
| | 10 | 7.75 | 0 |
| 32 | 1000 | 1/ 1.50 | 0 |
| | 100 | 2.00 | 0 |
| | 50 | 9.00 | 0 |
| 33 | 1000 | 24.00 | 0 |
| | 100 | 25.00 | 0 |
| | 50 | 25.00 | 0 |
| 33 | 1000 | 9.25 | 0 |
| | 100 | 21.00 | 0 |
| | 50 | 23.50 | 0 |
| 34 | 1000 | 1.00 | 0 |
| | 100 | 19.50 | 0 |
| | 50 | 19.50 | 0 |
| 34 | 1000 | 4.50 | 0 |
| | 100 | 10.50 | 0 |
| | 50 | 11.25 | 0 |
| 35 | 1000 | 2/ 1.25 | 0 |
| | 100 | 18.50 | 0 |
| | 50 | 22.25 | 0 |
| 36 | 100 | 0.00 | 0 |
| | 50 | 0.75 | 0 |
| | 25 | 2.25 | 0 |
| | 10 | 14.75 | 0 |

TABLE III (cont'd.)

| Compound of Example No. | Dosage ppm. | Mean Number of Mites per 6.28 sq. cm. | Plant Injury Rating |
|---|---|---|---|
| 37 | 1000 | 0.50 | 0 |
| | 100 | 8.25 | 0 |
| | 50 | 16.25 | 0 |
| 38 | 1000 | 0.00 | 0 |
| | 100 | 3.50 | 0 |
| | 50 | 20.50 | 0 |
| 39 | 1000 | 1.00 | 0 |
| | 100 | 4.75 | 0 |
| | 50 | 11.75 | 0 |
| 40 | 1000 | 18.25 | 0 |
| | 100 | 25.00 | 0 |
| | 50 | 22.50 | 0 |
| 40 | 4000 | 2.25 | 0 |
| | 2000 | 10.25 | 0 |
| | 1000 | 3.00 | 0 |
| 41 | 1000 | 0.00 | 0 |
| | 100 | 0.25 | 0 |
| | 50 | 9.50 | 0 |
| 41 | 100 | 14.25 | 0 |
| | 50 | 18.00 | 0 |
| | 25 | 25.00 | 0 |
| | 10 | 25.00 | 0 |
| 42 | 1000 | 3/ 22.00 | 0 |
| | 100 | 25.00 | 0 |
| | 50 | 25.00 | 0 |
| 42 | 1000 | 12.00 | 0 |
| | 100 | 23.50 | 0 |
| | 50 | 25.00 | 0 |

TABLE III (cont'd.)

| Compound of Example No. | Dosage ppm. | Mean Number of Mites per 6.28 sq. cm. | | Plant Injury Rating |
|---|---|---|---|---|
| 43 | 1000 | 0.00 | | 0 |
| | 100 | 12.75 | | 0 |
| | 50 | 21.75 | | 0 |
| 44 | 1000 | 0.00 | | 0 |
| | 100 | 7.25 | | 0 |
| | 50 | 18.50 | | 0 |
| 45 | 1000 | 0.00 | | 0 |
| | 100 | 0.50 | | 0 |
| | 50 | 3.25 | | 0 |
| 45 | 1000 | 14.25 | | - |
| | 100 | 22.00 | | - |
| | 50 | 24.50 | | - |
| 45 | 100 | 11.00 | | 0 |
| | 50 | 20.00 | | 0 |
| | 25 | 20.00 | | 0 |
| | 10 | 23.50 | | 0 |
| 46 | | nymphal and adult | larval | |
| | 1000 | 1.75 | 10.50 | 1.0 |
| | 100 | 0.00 | 6.25 | 0 |
| | 50 | 0.75 | 19.00 | 0 |
| | 25 | 2.00 | 14.00 | 0 |
| | 10 | 2.25 | 12.75 | 0 |
| 47 | 1000 | 0.00 | | 0.75 |
| | 100 | 25.00 | | 0 |
| | 50 | 25.00 | | 0 |
| 48 | 100 | 0.75 | | 0 |
| | 50 | 20.50 | | 0 |
| | 25 | 17.50 | | 0 |
| | 10 | 19.75 | | 0 |

TABLE III (cont'd.)

| Compound of Example No. | Dosage ppm. | Mean Number of Mites per 6.28 sq. cm. | | Plant Injury Rating |
|---|---|---|---|---|
| 48 | 1000 | 0.00 | | 0 |
| | 100 | 13.50 | | 0 |
| | 50 | 11.50 | | 0 |
| | 25 | 24.75 | | 0 |
| | 10 | 25.00 | | 0 |
| 48 | | treatment one day after infestation | treatment three days after infestation | |
| | 1000 | 0.25 | 0.00 | - |
| | 100 | 0.00 | 0.00 | - |
| | 50 | 0.50 | 0.00 | - |
| | 25 | 1.75 | 0.00 | - |
| | 10 | 2.00 | 0.00 | - |
| 49 | 4000 | 0.75 | | 0 |
| | 2000 | 0.00 | | 0 |
| | 1000 | 1.75 | | 0 |
| | 100 | 8.75 | | 0 |
| | 50 | 19.00 | | 0 |
| 49 | 4000 | 1.25 | | 0 |
| | 2000 | 4.25 | | 0 |
| | 1000 | 4.50 | | 0 |
| | 100 | 13.25 | | 0 |
| | 50 | 20.25 | | 0 |
| 50 | 1000 | 1.00 | | 0 |
| | 100 | 0.50 | | 0 |
| | 50 | 11.50 | | 0 |
| 51 | 1000 | 0.00 | | 0 |
| | 100 | 0.00 | | 0 |
| | 50 | 0.00 | | 0 |
| 52 | 1000 | 0.25 | | 0 |
| | 100 | 20.75 | | 0 |
| | 50 | 25.00 | | 0 |
| 53 | 1000 | 0.00 | | 0 |
| | 100 | 7.75 | | 0 |
| | 50 | 23.00 | | 0 |

TABLE III (cont'd.)

| Compound of Example No. | Dosage ppm. | Mean Number of Mites per 6.28 sq. cm. | Plant Injury Rating |
|---|---|---|---|
| 54 | 1000 | 0.00 | 2.75 |
| | 100 | 1.75 | 0 |
| | 50 | 17.75 | 0 |
| 55 | 1000 | 0.00 | 0 |
| | 100 | 3.00 | 0 |
| | 50 | 9.25 | 0 |
| 56 | 1000 | 0.00 | 0 |
| | 100 | 1.25 | 0 |
| | 50 | 9.25 | 0 |

1/ Control plants for this test (solvent conc. = 40,000 ppm) showed a mean of only 22.25 mites.

2/ Control plants for this test (solvent conc. = 40,000 ppm) showed a mean of only 19.25 mites.

3/ Control plants for this test (solvent conc. = 70,000 ppm) showed a mean of only 19.50 mites.

4/ Control plants for this test (solvent conc. = 100,000 ppm) showed a mean of only 21.75 mites.

5/ Formulated as an emulsifiable concentrate as described in Example 55; carried out at same time as test immediately preceding in this table for a comparison of formulation effect.

6/ Readings were made at 18 hours, 24 hours, 42 hours, 48 hours, 66 hours, 72 hours, and 90 hours after treatment of the plants.

EXAMPLE 59: FORMULATIONS

Various of the compounds to be employed in accordance with the present invention were formulated as emulsifiable concentrates and/or wettable powders. The composition of the formulations was as follows:

Emulsifiable Concentrate (2 lbs./gal., hereinafter "2EC")

| component | percent |
| --- | --- |
| N-methyl-2,4-difluoro-2',4'-dinitro-6'-(trifluoromethyl)diphenylamine | 25.0 |
| Toximul D | 5.0 |
| Toximul H | 5.0 |
| Dowanol EM | 20.0 |
| Xylene | 45.0 |

Wettable Powder (50%, hereinafter "50WP")

| component | percent |
| --- | --- |
| N-methyl-2,4-difluoro-2',4'-dinitro-6'-(trifluoromethyl)diphenylamine | 51.5 |
| Stepanol ME | 5.0 |
| Polyfon O | 5.0 |
| Zeolex-7 | 5.0 |
| Bardens Clay | 33.5 |

Emulsifiable Concentrate (1 lb./gal., hereinafter "1EC")

| component | percent |
| --- | --- |
| N-methyl-4-fluoro-2',4'-dinitro-6'-(trifluoromethyl)diphenylamine | 12.5 |
| Toximul D | 5.0 |
| Toximul H | 5.0 |
| Xylene | 77.5 |

Emulsifiable Concentrate (1 lb./gal., hereinafter "1EC")

| component | percent |
|---|---|
| N-methyl-3-(trifluoromethyl)-2',4'-dinitro-6'-(trifluoromethyl)diphenylamine | 12.5 |
| Toximul D | 5.0 |
| Toximul H | 5.0 |
| Xylene | 77.5 |

Emulsifiable Concentrate (0.5 lb./gal., hereinafter "0.5EC")

| component | percent |
|---|---|
| N-methyl-4-bromo-2',4'-dinitro-6'-(trifluoromethyl)diphenylamine | 6.5 |
| Toximul D | 5.0 |
| Toximul H | 5.0 |
| Dowanol EM | 20.0 |
| Xylene | 63.5 |

Emulsifiable Concentrate (1 lb./gal., hereinafter "1EC")

| component | percent |
|---|---|
| N-methyl-4-chloro-2',4'-dinitro-6'-(trifluoromethyl)diphenylamine | 12.5 |
| Toximul D | 5.0 |
| Toximul H | 5.0 |
| Acetophenone | 77.5 |

Emulsifiable Concentrate (1.5 lb./gal., hereinafter "1.5EC")

| component | percent |
|---|---|
| N-methyl-2,4-difluoro-2',4'-dinitro-6'-(trifluoromethyl)-diphenylamine | 19.0 |
| Toximul D | 2.5 |
| Toximul H | 2.5 |
| Dowanol EM | 22.0 |
| Xylene | 54.0 |

Emulsifiable Concentrate (1.5 lb./gal., hereinafter "1.5EC")

| component | percent |
|---|---|
| N-methyl-3-(trifluoromethyl)-2',4'-dinitro-6'-(trifluoromethyl)diphenylamine | 19.0 |
| Toximul D | 2.5 |
| Toximul H | 2.5 |
| Dowanol EM | 22.0 |
| Xylene | 54.0 |

EXAMPLE 60: COMPARATIVE PHYTOTOXICITY TESTS

As noted previously, the compounds to be employed in accordance with the present invention exhibit little or no phytotoxicity at use rates. This absence of phytotoxicity is surprising in view of the significant phytotoxicity of many diphenylamines of the prior art. These differences are illustrated by the following greenhouse evaluation for crop response of soybeans to various diphenylamines.

Each compound was formulated in a small amount of 50:50 ethanol:acetone and Tween 20, then diluted with water and separated into multiple formulations containing the respective compound at various concentrations. Because of preliminary indications of higher phytotoxicity of an N-H diphenylamine in comparison with its corresponding N-$CH_3$ diphenylamine (see Example 67 hereinabove), different concentrations were necessarily evaluated in an attempt to determine the highest concentration of the spray formulation free of phytotoxicity. Generally, N-H compounds were evaluated at 125, 62.5 and 31.25 ppm, and N-$CH_3$ compounds, at 4000, 2000 and 1000 ppm. Where necessary, selected compounds were retested at lower concentrations. Treated plants were held and observed for leaf distortion and leaf necrosis. However, any other kind of apparent response was noted. Each formulation was sprayed onto Kent-variety soybeans which were 21-days old (two fully-expended trifoliate leaves and a juvenile are present). There were two replicates per treatment, and each replicate consisted of two plants. Not all of the treatments were conducted at the same time, but each group of treatments included a solvent control and an untreated control.

The results of this evaluation for phytotoxicity were as set forth in the following table. Blanks in the table indicate that the compound in question was not tested. All data on a single line represents tests conducted at the same time. Neither the solvent check nor the untreated check exhibited any phytotoxicity in any of the tests.

TABLE XI

COMPARATIVE PHYTOTOXICITY TEST

| 2',4'-Dinitro-6'-(trifluoromethyl)-diphenylamine compound--substitution on ring | Highest concentration (ppm) free of phytotoxicity | | | |
|---|---|---|---|---|
| | N-H | $N-CH_3$ | $N-C_2H_5$ | $N-n-C_3H_7$ |
| 2-F | 62.5 | 4000 | | |
| 3-F | 31.25 | 4000[1)] | | |
| 3-F | 31.25 | 500 | | |
| 4-F | 31.25 | 2000 | | |
| 2,4-di F | 31.25 | 1000 | | |
| 2,4-di F | <31.25 | | 4000 | |
| 2,4-di F | 15.62 | 4000 | | |
| 2,5-di F | <31.25 | 2000 | | |
| 2,6-di F | 31.25 | 4000 | | |
| 3,4-di F | <31.25 | 1000 | | |
| 2,4,6-tri F | <31.25 | 4000 | | |
| 2,3,5,6-tetra F | <31.25 | 4000 | | |
| 2,3,4,5,6-penta F | <31.25 | 4000 | | |
| 2-Cl | <31.25 | 4000 | | |
| 3-Cl | <31.25 | 4000 | | |
| 4-Cl | <31.25 | 4000 | | |
| 3,5-di Cl | <31.25 | 4000 | | |
| 2,4,6-tri Cl | <31.25 | 1000 | | |
| 2,4,6-tri Cl | <31.25 | | 1000 | 1000 |
| 2,3,4,5,6-penta Cl | 62.5 | 4000 | | |
| 3-Br | <31.25 | 4000 | | |
| 4-Br | <31.25 | 4000 | | |
| 2,4-di Br | <31.25 | 4000 | | |
| 4-I | <31.25 | 4000 | | |

TABLE XI (cont'd.)

| 2',4'-Dinitro-6'-(trifluoromethyl)-diphenylamine compound--substitution on ring | Highest concentration (ppm) free of phytotoxicity | | | |
|---|---|---|---|---|
| | N-H | $N-CH_3$ | $N-C_2H_5$ | $N-n-C_3H_7$ |
| $3-CF_3$ | 7.81 | 4000 | | |
| $4-CF_3$ | <31.25 | <1000 | | |
| $4-CF_3$ | < 7.81 | 1000 | | |
| $2-Cl-5-CF_3$ | <31.25 | <1000 | | |
| $2-Cl-5-CF_3$ | 7.81 | 250 | | |

1/ Neither leaf distortion nor leaf necrosis was noted at any of the three rates tested; however, leaf chlorosis was noted and was rated 4 at 1000 ppm, 6 at 2000 ppm, and 6 at 4000 ppm (on a scale of 0-10 with 0 = no effect and 10 = severe effect).

The compounds to be employed in the above described insecticidal and arachnicidal uses also exhibit ectoparasiticidal activity when applied to the locus of ectoparasites on the surface of warm blooded animals. The compounds control ectoparasites by contact action.

CLAIMS

1. A method of controlling insects, arachnids, or ectoparasites which comprises applying to a locus of said insects, arachnids or ectoparasites a diphenylamine of formula (I):

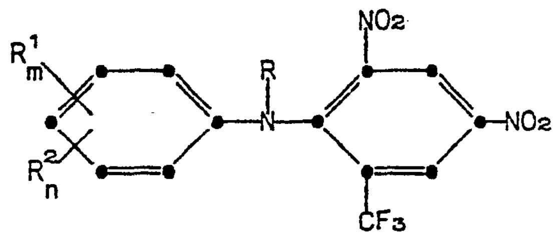

(I)

wherein

R is methyl, ethyl, or n-propyl;

each $R^1$ is independently bromo, chloro, or fluoro;

$R^2$ is iodo, cyano, nitro, or trifluoromethyl;

m is an integer of from 0 to 5;

n is an integer of from 0 to 1;

and the sum of m and n is an integer

(1) when n is 0, from 1 to 5; and

(2) when n is 1, from 1 to 3

subject to the further limitations that (1) not more than two $R^1$ groups represent bromo; (2) where $R^2$ is iodo, the iodo is located at the 4-position; (3) where $R^2$ is cyano, the cyano is located at the 3-, 4-, or 5-position; and (4) where $R^2$ is $NO_2$, the $NO_2$ is located at the 2-, 3-, 5-, or 6-position.

2. A method according to claim 1 for inhibiting phytophagous mites.

3. A method according to claim 1 or 2, wherein the diphenylamine is N-methyl-2,4-difluoro-2',4'-dinitro-6'-(trifluoromethyl)diphenylamine.

4. A method according to claim 1 or 2, wherein the diphenylamine is N-methyl-3-(trifluoromethyl)-2',4'-dinitro-6'-(trifluoromethyl)diphenylamine.

5. A diphenylamine of formula (I) as defined in claim 1 for use as a miticide.

6. A wettable powder or emulsifiable concentrate comprising as an active ingredient a diphenylamine of formula (I) as defined in claim 1.

7. A wettable powder or emulsifiable concentrate as claimed in claim 6, wherein the diphenylamine is N-methyl-3-(trifluoromethyl)-2',4'-dinitro-6'-(trifluoromethyl)diphenylamine.

8. An insecticidal, arachnicidal or ectoparasiticidal composition which comprises as an active ingredient: N-methyl-3-(trifluoromethyl)-2',4'-dinitro-6'-(trifluoromethyl)diphenylamine or N-methyl-2,4-difluoro-2',4'-dinitro-6'-(trifluoromethyl)diphenylamine, associated with at least one non-phytotoxic carrier.

9. A composition as claimed in claim 8, which comprises a surfactant.

10. A diphenylamine of formula (II):

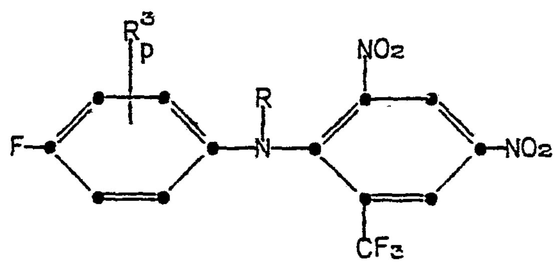

(II)

wherein R is methyl, ethyl, or n-propyl; and $R^3_p$ is one of the following:

European Patent Office

# EUROPEAN SEARCH REPORT

0060951

Application number

EP 81 30 5139

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
| --- | --- | --- | --- |
| D,Y | US - A - 4 187 318 (B.A. DREIKORN) * column 1, line 67 - column 2, line 53; examples; column 14, line 7 - column 17, line 1 * --- | 1-12 | C 07 C 87/62<br>121/78<br>A 01 N 33/18<br>37/34 |
| Y | GB - A - 1 548 702 (ELI LILLY & COMP.) * page 1, line 24 - page 2, line 7; page 2, line 26 - page 3, line 43; example * --- | 1-12 | |
| D,Y | US - A - 4 117 167 (C.B. BARLOW) * column 1, line 16 - column 6, line 56 * --- | 1-12 | TECHNICAL FIELDS SEARCHED (Int. Cl. 3) |
| A | US - A - 2 212 825 (H.W. DAUDT) * example 11 * --------- | 10 | C 07 C 87/00<br>A 01 N 33/00 |

The present search report has been drawn up for all claims

| Place of search | Date of completion of the search | Examiner |
| --- | --- | --- |
| The Hague | 14-06-1982 | PAUWELS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document